# EUROPÄISCHE PATENTSCHRIFT

(11) **EP 1 616 964 B1**
(45) Veröffentlichungstag und Bekanntmachung des Hinweises auf die Patenterteilung: **09.12.2009**
(21) Anmeldenummer: 05090183.4
(22) Anmeldetag: 22.06.2005
(51) Int. Cl.: C12P 21/02

(54) **Verfahren zur präparativen in vitro Proteinbiosynthese**
Process for preparative in vitro protein biosynthesis
Procédé préparatif pour la biosynthèse de protéines in vitro

(30) Priorität: 30.06.2004 DE 102004032460
(43) Veröffentlichungstag der Anmeldung: 18.01.2006
(73) Patentinhaber: QIAGEN GmbH, 40724 Hilden (DE)
(72) Erfinder: Strey, Jan, Dr., 14195 Berlin (DE); Merk, Helmut, Dr., 14195 Berlin (DE); Stiege, Wolfgang, Dr., 14195 Berlin (DE)
(74) Vertreter: Jungblut, Bernhard Jakob

(56) Entgegenhaltungen:
- EP-A1- 0 312 617
- KIM D-M ET AL: "PROLONGING CELL-FREE PROTEIN SYNTHESIS BY SELECTIVE REAGENT ADDITIONS" BIOTECHNOLOGY PROGRESS, Bd. 16, 2000, Seiten 385-390, XP002928933 ISSN: 8756-7938
- KIGAWA T ET AL: "Cell-free production and stable-isotope labeling of milligram quantities of proteins" FEBS LETTERS, ELSEVIER, AMSTERDAM, NL, Bd. 442, Nr. 1, 8. Januar 1999 (1999-01-08), Seiten 15-19, XP004258994 ISSN: 0014-5793

## Beschreibung

### Gebiet der Erfindung

Die Erfindung betrifft ein Verfahren zur in vitro Proteinsynthese in einem zellfreien Transkriptions-/Translationssystem mit den folgenden Verfahrenstufen: a) es wird in einem Reaktionsgefäß eine Reaktionslösung mit den folgenden Synthesesubstanzen angesetzt: Komponenten des Transkriptions-/Translationsapparates für ein definiertes Protein, Aminosäuren, sowie Energie liefernde und für die Synthese des definierten Proteins notwendige Stoffwechselbestandteile, b) in dem Reaktionsgefäß wird die Synthese in einem definierten Zeitraum durchgeführt, c) nach Ablauf des definierten Zeitraumes wird die Reaktionslösung einer Abtrennungsverfahrensstufe unterworfen, in welcher gebildete Stoffwechselprodukte aus der Lösung abgetrennt (und gewonnen) werden. Der Begriff der Proteinsynthese bezeichnet im Rahmen der Erfindung die Expression des Proteins.

### Stand der Technik

Verfahren zur zellfreien Expression von Proteinen sind beispielsweise aus den Literaturstellen EP 0312 617 B1, EP 0401 369 B1 und EP 0 593 757 B1 bekannt.

Demgemäß werden die für eine Transkription und/oder Translation notwendigen Komponenten zusammen mit einem für ein gewünschtes Protein kodierenden Nukleinsäurestrang in einem Reaktionsgefäß inkubiert und nach der Expression die Polypeptide/Proteine aus der Reaktionslösung isoliert.

Sowohl die für die Transkription, als auch die für die Translation notwendigen Komponenten lassen sich leicht aus den Überständen pro- oder eukaryontischer Zelllysate nach einer 30000 x g oder 10000 x g Zentrifugation gewinnen. Dieser sogenannte S-30 bzw. S-10 Extrakt enthält alle die für die Transkription und Translation notwendigen Komponenten außer niedermolekulare Komponenten.

In den meisten Fällen befindet sich das Gen, bzw. der für das Protein kodierende Nukleinsäurestrang, unter der Kontrolle eines T7-Promotors. Dies hat zum Vorteil, daß durch den Einsatz von Rifampicin vorhandene *E.coli* RNA-Polymerase inhibiert werden kann, und somit etwaige aus dem S30-Extrakt oder aus der Vektorpräparation stammende endogene E.coli DNA nicht transkribiert wird. Wird dagegen ein unter einem E.coli Promotor stehendes Gen exprimiert, kann, sofern nicht bereits im Extrakt vorhanden, eine E.coli Polymerase eingesetzt werden, was zu einer Co-expression etwaiger endogener E.coli DNA und somit zu ungewünschten, endogenen Proteine führen kann. Die Expression erfolgt typischerweise bei 37°C, kann aber auch bei Temperaturen von 17°C bis 45°C erfolgen. Die Anpassung der Temperatur empfiehlt sich insbesondere bei der Expression von Proteinen, in denen eine komplexe Sekundär-/Tertiärstruktur ausgebildet werden soll. Durch Absenken der Temperatur kann die Syntheserate gesenkt und somit den Proteinen die Möglichkeit gegeben werden, sich richtig zu falten, um ein funktionstüchtiges/aktives Protein zu erhalten. Auch kann auf die Ausbildung von Disulfidbrücken innerhalb der exprimierten Proteine Einfluß über das Reduktionspotetial der Reaktionslösung durch den Zusatz von z.B. Dithiothreitol (DTT) und/oder oxidiertem/reduziertem Glutathion genommen werden.

Vor jeder neuen Proteinsynthese sollten die jeweiligen Systeme idealerweise einer Optimierung unterzogen werden. Dabei werden die Konzentrationen an zweiwertigen Magnesium-Ionen (Mg²⁺), an RNA/DNA-Polymerase und dem als Matrize dienenden kodierenden Nukleinsäurestrang variiert.

In dem in dem Dokument EP 0312 617 B1 offenbarten Verfahren zur zellfreien Expression von Proteinen wird der für das Protein kodierende Nukleinsäurestrang als mRNA der Reaktionslösung zugegeben. Damit müssen für die Herstellung von Polypeptiden in dem zellfreien System nur die für die Translation notwendigen Komponenten des Translationsapparates, insbesondere Ribosomen, Initiations-, Elongations-, Freisetzungsfaktoren und Aminoacyl-tRNA-Synthetasen, sowie Aminosäuren und als Energie liefernde Substanzen ATP und GTP in ein Reaktionsgefäß gegeben werden. Bei der anschließenden Polypeptid-/Proteinsynthese kommt es neben der Bildung von Polypeptiden/Proteinen auch zur Bildung von niedermolekularen Substanzen, wie ADP, AMP, GDP, GMP und anorganischen Phosphaten unter Verbrauch der Energie liefernden Substanzen ATP und GTP und von Aminosäuren. Diese führt dazu, daß die Reaktion nach dem Verbrauch von ATP oder GTP oder einer Aminosäure, bzw. durch die als Inhibitoren wirkenden gebildeten niedermolkularen Substanzen nach einiger Zeit zum Erliegen kommt. Um dem entgegen zu wirken, offenbart die Literaturstelle EP 0312 617 B1 daß die während der Translation verbrauchten Substanzen während der Translation herausgeführt und gleichzeitig die Energie liefernden Substanzen und die Aminosäuren zur Erhaltung der Ausgangskonzentration eingeführt werden.

Demgegenüber offenbart das Dokument EP 0401 369 B1 ein Verfahren, bei dem die für das Protein kodierende Nukleinsäurestrang als mRNA oder DNA der Reaktionslösung zugegeben werden kann. Letzteres hat den Vorteil, daß DNA wesentlich stabiler als mRNA ist, und der notwendige Umschreibeprozess der DNA in RNA vor der Reaktion nicht notwendig ist, sondern gleich die DNA, z.B. als Vektor oder lineares Konstrukt eingesetzt werden kann. Durch den Einsatz der DNA muß das zellfreie Expressionssystem neben den obengenannten Translationsfaktoren noch die für die Transkription der DNA in RNA notwendigen Transkriptionsfaktoren, wie z.B. RNA-Polymerase, Sigma-Faktor und Rho-Protein und die Nukleotide ATP, UTP, GTP und CTP enthalten. Auch hier müssen die während der Transkription/Translation verbrauchten niedermolekularen Substanzen, wie ADP, AMP, GDP, GMP und anorganische Phosphate, während der Translation herausgeführt werden und gleichzeitig die Energie liefernde Substanzen, Nukleotide und die Aminosäuren zur Erhaltung der Ausgangskonzentration eingeführt werden. Aus dem Dokument EP 0 593 757 B1 ist es bekannt, neben den verbrauchten niedermolekularen Substanzen auch die exprimierten Polypeptide durch eine Ultrafiltrationsbarriere während der Translation aus der Reaktionslösung abzutrennen. Es handelt sich bei diesen Verfahren folglich um kontinuierliche Syntheseverfahren.

Bei den kontinuierlichen Syntheseverfahren sind die erzielten langen Reaktionszeiten zwar an sich für die Ausbeute vorteilhaft, hiermit sind jedoch auch wiederum andere Nachteile verbunden. Zum ersten wird die Qualität der neusynthetisierten Proteine durch die lange Verweilzeit negativ beeinflusst, beispielsweise auf Grund von Degradation, (Nach-) Präzipitation, oder - bei Verwendung von Isotopen-markierten Aminosäuren - unerwünschter Verteilung der Isotope auf andere Aminosäurenspezies (bedingt durch den Aminosäuremetabolismus). Zum anderen muss bei der (kontinuierlichen) Zufuhr verbrauchter Substanzen mit Transportgradienten über Membranen und dergleichen gearbeitet werden, wofür die teuren niedermolekularen Substanzen, wie Energiekomponenten, in relativ hohen Mengen eingesetzt werden müssen.

Aus der Praxis sind Batchverfahren zur zellfreien Proteinbiosynthese bekannt, wobei während der Reaktionsdauer weder Produktproteine abgetrennt noch verbrauchte Substanzen zugeführt werden. Zwar ist die anfängliche Kinetik schnell, die Laufzeit ist jedoch kurz, so dass nur relativ wenig Protein erhalten wird. Daher werden solche Batchverfahren nur für analytische und nicht für präparative Zwekke eingesetzt.

Kim D-M et al (BIOTECHNOLOGY PROGRESS, Bd. 16, 2000, Seiten 385-390) offenbart ein zellfreies "Fed-Batch"-Expressionssystem. Dabei finden während der Inkubation wiederholte Zugaben von labilen Substraten statt, jedoch ohne Entfernung von niedermolekularen Stoffwechselprodukte und/oder Reaktionsinhibitoren.

Kigawa T. et al (FEBS Letters Bd. 442, Nr. 1, 8. Januar 1999, Seiten 15-19) beschreibt ein auf eine Dialysekammer beruhendes zellfreies Expressionssystem für Proteine. Die benötigten Substrate werden dabei über die frische Reaktionslösung in der Dialysekammer ersetzt, die sich bildenden Abbauprodukte werden jedoch auch während der Syntheseschritte entfernt.

### Technisches Problem der Erfindung

Der Erfindung liegt das technische Problem zugrunde, ein Verfahren zur präparativen in vitro Proteinsynthese anzugeben, welches hohe Ausbeuten bei schneller Kinetik (hohe Produktivität) bei gleichzeitig hoher Qualität der exprimierten Proteine und gegenüber kontinuierlichen Systemen reduziertem Einsatz teurer (Energie-) Komponenten gewährleistet.

Grundzüge der Erfindung und bevorzugte Ausführungsformen.

Zur Lösung dieses technischen Problems lehrt die Erfindung ein Verfahren zur präparativen in vitro Synthese eines Expressionsproduktes in einem zellfreien Transkriptions/Translationssystem mit den folgenden Verfahrensstufen: a) es wird in einem Reaktionsgefäß eine Reaktionslösung mit den folgenden Synthesesubstanzen angesetzt: Komponenten des Transkriptions-/Translationsapparates für ein definiertes Expressionsprodukt, Aminosäuren, sowie Energie liefernde und für die Synthese des definierten Proteins notwendige Stoffwechselbestandteile, b) in dem Reaktionsgefäß wird die Synthese in einem definierten Zeitraum durchgeführt, und zwar ohne Abtrennung von sich bildenden Substanzen und ohne Zufuhr von sich verbrauchenden Synthesesubstanzen innerhalb des definierten Zeitraumes, c) nach Ablauf des definierten Zeitraumes wird die Reaktionslösung einer Abtrennungsverfahrensstufe unterworfen, in welcher gebildete niedermolekulare Stoffwechselprodukte und/oder Reaktionsinhibitoren aus der Lösung abgetrennt werden, d) unmittelbar vor, nach oder zugleich mit Stufe c) werden verbrauchte Synthesesubstanzen supplementiert, e) die Stufen b), c) und d) werden mit der Reaktionslösung aus Stufe d) zumindest einmal wiederholt, wobei bei letztmaliger Ausführung der Stufe b) die Stufen c) und d) entfallen können.

Als Expressionsprodukte kommen insbesondere Proteine in Frage. Reaktionsinhibitoren sind Substanzen, die in der Reaktionslösung enthalten sind und/oder die während der Synthese gebildet werden und die Reaktionsgeschwindigkeit (Kinetik der Synthese) verlangsamen bzw. die Synthese vollständig unterbinden, verglichen mit einer Reaktionslösung ohne die Reaktionsinhibtoren. Der Begriff der Reaktionsinhibitoren im Sinne der Erfindung umfasst aber auch aus sonstigen Gründen unerwünschte Komponenten.

Grundsätzlich ist die mit der letztmaligen Ausführung der Stufe c) erhaltene Lösung bereits für verschiedene Zwecke, beispielsweise für analytische Zwecke, verwendbar. Wenn jedoch das Expressionsprodukt in aufgereinigter Form benötigt wird, kann dieses in der Stufe c) oder anschließend an Stufe e) aus der Lösung abgetrennt werden. Dies kann beispielsweise unter Verwendung einer mobilen oder immobilisierten Matrix erfolgen. Die Funktionsweise einer solchen Matrix kann auf allen für die Bindung von Expressionsprodukten bekannten Reinigungsmethoden, wie z.B. Ionentauscher, Affinität, Antigen/Antikörper-Wechselwirkung, Hydrophobe/Hydrophile-Wechselwirkung beruhen. Hierbei sind die geeigneten Moleküle an der Oberfläche eines Substrates gebunden. Für eine besonders effiziente Abtrennung können diese mit einem oder mehreren Markern, z.B. in Form von mehreren N- oder C-terminalen aufeinander folgenden Histidinen, oder einem oder mehreren anderen Proteinen, z.B. Glutathion, als ein sogenanntes Fusionsprotein koexprimiert werden. Die Matrix weist dann einen für diesen Marker/dieses Protein spezifische Bindungspartner auf, der eine effiziente Bindung des chimären Fusionsproteins über den Marker/den Fusionpartner an die Matrix ermöglicht. Die Matrix kann Anionen- oder Kationenaustauschermaterial oder Hydroxylapatit enthalten. Wenn die Proteine als Fusionsproteine exprimiert werden und die Fusionspartner N-, C-terminal oder innerhalb des exprimierten Proteins liegen, kann eine Matrix verwendet werden, die spezifisch den Fusionspartner bindet. So kann das Protein N- oder C-terminal mehrere aufeinander folgende Histidine, insbesondere 3 bis 12, vorzugsweise 5 bis 9, höchst vorzugsweise 6, enthalten, wobei die Matrix dann eine Metall-Chelat-Verbindung tragen kann, insbesondere mit zweiwertigen Metall-Ionen, vorzugsweise zweiwertige Kupfer- und/oder Nickel-Ionen. Das Protein kann N- oder C-terminal als Fusionspartner Gluthation-S-Transferase enthalten, wobei dann an die Matrix Glutathion gekoppelt sein kann. Das Protein kann eine Aminosäuresequenz enthalten, die es ihm ermöglicht an Streptavidin zu binden, vorzugsweise die Aminosäuresequenz AWRHPQFGG, höchstvorzugsweise die Aminosäuresequenz WSHPQFEK, wobei dann an die Matrix Streptavidin gekoppelt sein kann.

Grundsätzlich sind die einsetzbaren Komponenten aus dem Stand der Technik bekannt. Der Translationsapparat umfasst insbesondere Ribosomen, Initiations-, Elongations-, Freisetzungsfaktoren und Aminoacyl-tRNA-Synthetasen. Hiermit (und mit den weiteren Komponenten) kann die Translation von für ein zu synthetisierendes Protein codierender mRNA erfolgen. Bei Einsatz von für das zu synthetisierende Protein codierender DNA sind zusätzlich Transkriptionsfaktoren für die Transkription der DNA in RNA notwendig, wie z.B. RNA-Polymerase, Sigma-Faktor und Rho-Protein und die Nukleotide ATP, UTP, GTP und CTP. Die notwendigen Stoffwechselbestandteile der Reaktion sind aus der nicht abschließenden Gruppe "ATP, UTP, GTP und CTP, Pyrophosphat, Aminosäuren und Mischungen dieser Substanzen" ausgewählt. Eingesetzte Aminosäuren können natürliche Aminosäuren, aber auch chemisch derivatisierte nicht-natürliche Aminosäuren oder Isotopen-markierte Aminosäuren sein. Niedermolekulare Stoffwechselprodukte, die in der Recyclingstufe c) teilweise oder insgesamt (bezogen sowohl auf eine Stoffwechselproduktspezies als auch auf die Gesamtheit der Stofwechselprodukte) abgetrennt oder reduziert werden, sind beispielsweise ADP, AMP, GDP, GMP und anorganisches Phosphat. Niedermolekulare Stoffwechselprodukte haben ein Molekulargewicht von weniger als 10000 Da, vorzugsweise weinger als 8000 Da, höchstvorzugsweise weniger als 5000 Da. Sie können ein Molekulargewicht oberhalb 2000 Da aufweisen.

Eine Zugabe verbrauchter Synthesesubstanzen vor der Abtrennungsverfahrensstufe ist in den Fällen machbar, in welchen es sich bei den Synthesesubstanzen um hochmolekulare Synthesesubstanzen handelt. Diese haben Molekulargewichte, bei welchen die Molekulargewichte der niedermolekularen Stoffwechselprodukte, wie vorstehend angegeben, überschritten werden.

Das erfindungsgemäße Verfahren kann grundsätzlich sowohl mit prokaryontischen Systemen als auch in eukaryontischen Systemen durchgeführt werden. Die für die Transkription/Translation notwendigen Komponenten lassen sich leicht aus den Überständen pro- oder eukaryontischer Zelllysate beispielsweise nach einer 30000 x g oder 10000 x g Zentrifugation gewinnen. Dieser sogenannte S-30 bzw. S-10 Extrakt enthält alle die für die Transkription und Translation wesentlichen Komponenten.

Die Stufen b), c) und d) können einmal bis zehnmal, vorzugsweise einmal bis fünfmal wiederholt werden. Der definierte Zeitraum kann zwischen 0,1 bis 10 Stunden, vorzugsweise zwischen 0,5 bis 3 Stunden, betragen. Die Stufe c) kann mittels Gelfiltration, Ultrafiltration, Dialyse, Diafiltration oder Matrices mit selektiven Bindungseigenschaften für niedermolekulare Stoffwechselprodukte und/oder Reaktionsinhibitoren durchgeführt werden. Die Methoden der Gelfiltration, Ultrafiltration, Dialyse und Diafiltration sind dem Fachmann wohl vertraut. Beispielsweise zur Abtrennung von Phosphat kommen als Matrices Sevelamer® HCl oder Renagel® in Frage. Für Reaktionsinhibitoren kommen Matrices in Frage, die diese Reaktionsinhibitoren selektiv binden, wobei die vorstehenden Ausführungen zur Abtrennung von Expressionsprodukten analog zutreffen.

Das erfindungsgemäße Verfahren ist im Kern ein repetitives Batch Verfahren, wobei ein Batch Ansatz mit der gleichen Reaktionslösung wiederholt wird nach einer zwischengeschalteten Recyclingstufe, in welcher aus der Reaktionslösung niedermolekulare Stoffwechselprodukte abgetrennt und verbrauchte Substanzen nachgegeben werden. Mit der Erfindung werden einerseits gegenüber kontinuierlichen Verfahren verkürzte Reaktionszeiten erzielt. Dies resultiert in einer verbesserten Qualität des Produktproteins. Zudem müssen vergleichsweise weniger niedermolekulare Substanzen, insbesondere Energielieferanten, eingesetzt werden, da Konzentrationsgradienten nicht notwendig sind. Nur eine Supplementierung, i.e. eine Zugabe bis zum Erreichen einer definierten Anfangskonzentration, ist erforderlich. Trotzdem werden hohe Ausbeuten bei schnellen Kinetiken und folglich hohe Produktivitäten erhalten.

Eine Variante der Erfindung von selbstständiger Bedeutung betrifft ein Verfahren zur präparativen in vitro Proteinsynthese in einem zellfeien Transkriptions-/Translationssystem mit den folgenden Verfahrenstufen: a') es wird in einem Reaktionsgefäß eine Reaktionslösung mit den folgenden Synthesesubstanzen angesetzt: Komponenten des Transkriptions-/Translationsapparates für ein definiertes erstes Expressionsprodukt, optional Komponenten des Transkriptions-/Translationsapparates für ein definiertes von dem ersten Expressionsprodukt verschiedenes zweites Expressionsprodukt, Aminosäuren, sowie Energie liefernde und für die Synthese notwendige Stoffwechselbestandteile, b') in dem Reaktionsgefäß wird die Synthese des ersten Proteins in einem ersten definierten Zeitraum durchgeführt, und zwar ohne Zufuhr von sich verbrauchenden Synthesesubstanzen innerhalb des ersten definierten Zeitraumes, sowie c') optional werden gebildete niedermolekulare Stoffwechselprodukte aus der Lösung abgetrennt, d') nach Ablauf des definierten Zeitraumes wird die Reaktionslösung mit verbrauchten Synthesesubstanzen supplementiert und, sofern nicht bereits in Stufe a) zugesetzt, mit Komponenten des Transkriptions-/Translationsapparates für das definierte zweite Expressionsprodukt versetzt, e') in dem Reaktionsgefäß wird die Synthese des zweiten Proteins in einem definierten zweiten Zeitraum durchgeführt, und zwar ohne Abtrennung von sich bildenden Substanzen und ohne Zufuhr von sich verbrauchenden Synthesesubstanzen innerhalb des definierten Zeitraumes.

Anschließend kann das Expressionsprodukt aus der Lösung abgetrennt werden, die das Expressionsprodukt enthaltende Lösung kann aber direkt auch für andere Zwecke, beispielsweise Analytik oder im Rahmen des Screenings einer Substanzbibliothek ohne eine solche Abtrennung unmittelbar weiter verwendet werden. Grundsätzlich kann sich aber auch das Verfahren der Ansprüche 1 bis 5, beginnend mit dessen Stufe c), anschließen. Stufe e) kann dabei entfallen. Es gelten die zum Verfahren nach einem der Ansprüche 1 bis 5 getroffenen Ausführungen analog.

In dieser Variante der Erfindung sind verschiedene "Programmierungen" möglich. Hiermit ist gemeint die Art und Weise der Steuerung der Synthese der verschiedenen Expressionsprodukte in den verschiedenen Stufen.

Die Transkriptions-/Translationsapparate für das erste Expressionsprodukt und das zweite Expressionsprodukt können verschiedene erste und zweite regulatorische Sequenzen enthalten, wobei eine für das erste Expressionsprodukt codierende erste Gensequenz unter der Kontrolle der ersten regulatorischen Sequenz und eine für das zweite Expressionsprodukt codierende zweite Gensequenz unter der Kontrolle der zweiten regulatorischen Sequenz stehen.

In der Ausführungsform mit Komponenten des Transkriptions-/Translationsapparates für ein definiertes von dem ersten Expressionsprodukt verschiedenes zweites Expressionsprodukt in Stufe a') kann in der Stufe b') die zweite regulatorische Sequenz inhibiert werden, und kann in der Stufe e') die erste regulatorische Sequenz inhibiert werden. In der Ausführungsform mit Zugabe der Komponenten des Transkriptions-/Translationsapparat für das definierte zweite Expressionsprodukt in Stufe d'), kann in der Stufe e') die erste regulatorische Sequenz inhibiert werden.

In dieser Variante der Erfindung wird ebenfalls ein repetitives Batchverfahren angewandt, wobei jedoch verschiedene Expressionsprodukte, beispielsweise Proteine, in verschiedenen Stufen erhalten werden. Dabei ist das zweite Expressionsprodukt typischerweise das tatsächlich gewünschte Produktprotein. Das erste Expressionsprodukt ist dagegen eine Hilfssubstanz, wie beispielsweise Translationsfaktoren, Faltungshelferproteine, Interaktionspartner, oder tRNA. Solche Substanzen sind der Bildung des Produktproteins förderlich, beispielsweise hinsichtlich der Ausbeute, Löslichkeit oder Funktionalität. In Frage kommen als erste Expressionsprodukte beispielsweise Chaperone, die der Löslichkeit des Produktproteins förderlich sind. Insofern bezeichnet der Begriff der Synthese auch den Begriff der Reifung eines Proteins.

Grundsätzlich kann die Lösung in der Stufe c) bzw. c') auch aufkonzentriert werden, beispielsweise durch Dialyse gegen eine PEG Lösung.

Im Folgenden wird die Erfindung anhand von lediglich Ausführungsformen darstellenden Beispielen näher erläutert.

### Beispiel 1: einfache Repetition mit einer Recyclingstufe

0,5 ml einer Reaktionslösung für die zellfreie Proteinbiosynthese, enthaltend 175 µl S-Mix, 150 µl T-Mix, 40 µl E-Mix (erhältlich als Komponenten des "RiNA in-vitro PBS Kits", Kat.-Nr. P-1102-14, RiNA GmbH, Berlin, Deutschland) 63 µM ¹⁴C-Leucin (100 dpm/pmol), 5 nM Plasmid DNA, codierend für den Elongationsfaktor Ts aus E. coli, und RNase freies Wasser ad 0,5 ml, wurden nach Inkubation (1,5 h, 37°C) mittels Ultrafiltration (10 kDa-Membran) auf 50% eingeengt (250 µl) und anschließend mit 250 µl Supplementierungsmix folgender Zusammensetzung versetzt: 100 mM HEPES (pH 7,6), 200 mM Kaliumacetat, 100 mM Ammoniumacetat, 46 mM Magnesiumchlorid, 0,2 mM EDTA, 0,04% Natriumazid (w/v), 10 mM DTT, 20 µM GDP, 8% PEG3000 (w/v), 200 µM Folsäure, je 1,2 mM aller 20 Aminosäuren, 126 µM ¹⁴C-Leucin, je 2 mM ATP und GTP, je 1 mM UTP und CTP, 60 mM Phosphoenolpyruvat und 20 mM Acetylphosphat. Die nachfolgende zweite Synthese erfolgte für 1,5 h bei 37°C. Die erhaltenen Mengen an EF-Ts sind (in toto) nach der ersten Synthese 114 µg und nach der zweiten Synthese 221 µg. Die Quantifizierung erfolgte durch Bestimmung des Einbaus von eingesetztem radioaktiv markiertem ¹⁴C-Leucin.

### Beispiel 2: vierfache Repetition einer Batch-Reaktion mit vier Recyclingstufen.

1 ml einer Reaktionslösung für die zellfreie Proteinbiosynthese, enthaltend 350 µl S-Mix, 80 µl E-Mix (erhältlich als Komponenten des "RiNA in-vitro PBS Kits", Kat.-Nr. P-1102-14, RiNA GmbH, Berlin, Deutschland), 35 mM HEPES (pH 7,6), 70 mM Kaliumacetat, 35 mM Ammoniumacetat, 10 mM Magnesiumchlorid, 0,07 mM EDTA, 0,014% Natriumazid (w/v), 5 mM DTT, 100 µM Folsäure, je 1,2 mM aller 20 Aminosäuren, 63 µM ¹⁴C-Leucin, 5 nM Plasmid DNA, codierend für den Elongationsfaktor Ts aus E. coli, und RNase freies Wasser ad 1 ml, wurden nach Inkubation (1,5 h, 37°C) über eine Sephadex-Matrix (G-25) gelfiltriert, mittels Ultrafiltration (10 kDa-Membran) auf 50% des Ausgangsvolumens eingeengt (500 µl) und anschließend mit 500 µl Supplementierungsmix folgender Zusammensetzung versetzt: 100 mM HEPES (pH 7,6), 200 mM Kaliumacetat, 100 mM Ammoniumacetat, 26 mM Magnesiumchlorid, 0,2 mM EDTA, 0,04% Natriumazid (w/v), 10 mM DTT, 20 µM GDP, 200 µM Folsäure, je 2,4 mM aller 20 Aminosäuren, 126 µM ¹⁴C-Leucin, je 2 mM ATP und GTP, je 1 mM UTP und CTP, 60 mM Phosphoenolpyruvat und 20 mM Acetylphosphat. Die nachfolgende zweite Synthese erfolgte für 1,5 h bei 37°C. Die Recyclingstufe (Gelfiltration, Ultrafiltration, Supplementierung) und die Synthesestufe wurden anschließend mehrfach wiederholt (Recycling: noch dreimal = insgesamt viermal; Synthese: noch dreimal = insgesamt fünfmal). Die erhaltenen Mengen an EF-Ts sind (in toto) nach der ersten Synthese 171 µg, nach der zweiten Synthese 315 µg, nach der dritten Synthese 447 µg, nach der vierten Synthese 561 µg und nach der fünften Synthese 650 µg. Die Quantifizierung erfolgte durch Bestimmung des Einbaus von eingesetztem radioaktiv markiertem ¹⁴C-Leucin.

### Beispiel 3: zweifache Repetition einer high yield Batch Reaktion mit zwei Recyclingstufen

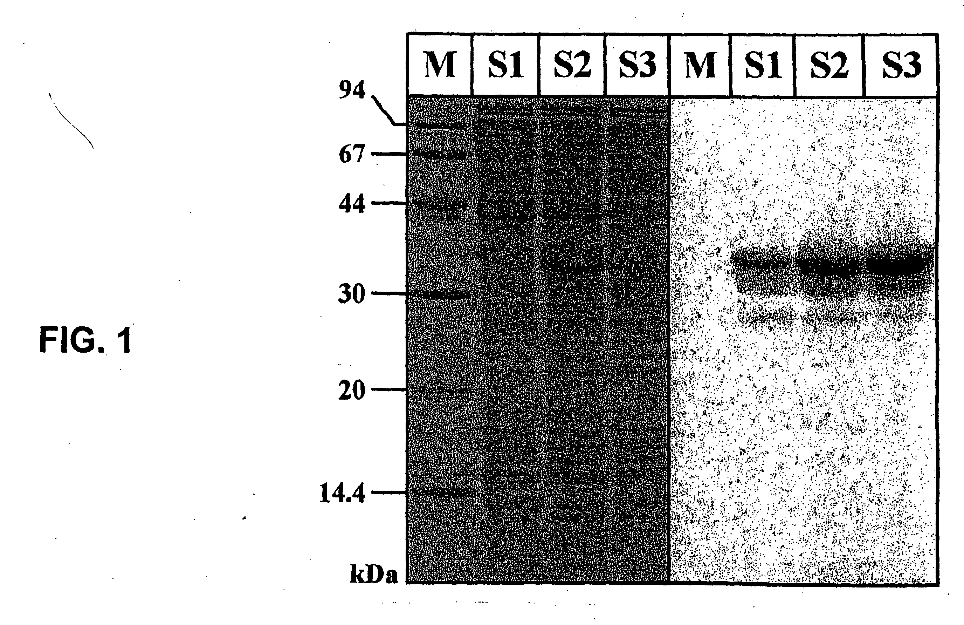

1,8 ml einer Reaktionslösung für die zellfreie Proteinbiosynthese wurden wie folgt präpariert: 720 µl EasyXPress® Reaction Buffer wurden mit 630 µl E. coli Extrakt (beide Komponenten enthalten im EasyXPress® Protein Synthesis Maxi Kit, Kat.-Nr. 32506, Qiagen GmbH, Hilden, Deutschland,) 63 µM ¹⁴C-Leucin (100 dpm/pmol), 10 mM Plasmid DNA, codierend für den Elongationsfaktor Ts aus E. coli, und RNase-freiem Wasser ad 1,8 ml versetzt. Die Reaktion wurde anschließend mittels Ultrafiltration (10 kDa-Membran auf 1 ml aufkonzentriert und 1 h bei 37°C inkubiert. Nach dieser ersten Synthesephase wurde der Ansatz über eine Nap-10 Säule (Sephadex G-25) gelfiltriert und mit 300 µl einer Lösung enthaltend 300 mM HEPES (pH 7,6), 600 mM Kaliumacetat, 300 mM Ammoniumacetat, 114 mM Magnesiumchlorid, 0,6 mM EDTA, 0,12% Natriumazid (w/v), 6 mM DTT, 60 µM GDP, 24% PEG3000 (w/v), 600 µM Folsäure, je 7,2 mM aller 20 Aminosäuren, 380 µM ¹⁴C-Leucin, je 10,2 mM ATP und GTP, je 5,1 mM UTP und CTP, 306 mM Phosphoenolpyruvat und 102 mM Acetylphosphat, supplementiert. Die nachfolgende zweite Synthese erfolgte für 1,0 h bei 37°C. Im Anschluss daran wurden die Recyclingstufe (Gelfiltration, Supplementierung) und die Synthesestufe ein weiteres Mal wiederholt, wobei der Supplementierungsmix nunmehr die folgende Zusammensetzung hatte: 300 mM HEPES (pH 7,6), 600 mM Kaliumacetat, 300 mM Ammoniumacetat, 78 mM Magnesiumchlorid, 0,6 mM ED-TA, 0,12% Natriumazid (w/v), 6 mM DTT, 60 µM GDP, 24% PEG3000 (w/v), 600 µM Folsäure, je 7,2 mM aller 20 Aminosäuren, 380 µM ¹⁴C-Leucin, je 6 mM ATP und GTP, je 3 mM UTP und CTP, 180 mM Phosphoenolpyruvat und 60 mM Acetylphosphat. Insgesamt wurden also 3 Synthesestufen und 2 Recyclingstufen durchlaufen. Die erhaltenen Mengen an EF-Ts sind (in toto) nach der ersten Synthese 563 µg, nach der zweiten Synthese 1804 µg und nach der dritten Synthese 2487 µg. Durch zweimalige Wiederholung wird also das 4,4-fache der ersten Synthesestufe an Ausbeute erhalten. Die Quantifizierung erfolgte durch Bestimmung des Einbaus von eingesetztem radioaktiv markiertem ¹⁴C-Leucin. Figur 1 zeigt eine SDS-PAGE-Analyse des Produktproteins aus diesem Beispiel. Auf der linken Seite ist die Coomassie-Färbung erkennbar und auf der rechten Seite das Autoradiogramm. Die Bahn M ist der Molekulargewichtsstandard, die Bahnen S1 bis S3 sind die 3 Synthesestufen. Die theoretische Größe des EF-Ts beträgt 31,6 kDa.

### Beispiel 4: Programmierung/Konditionierung eines Translationssystems

In einer ersten Synthesestufe wird ein Gen für ein die Synthese bzw. Qualität des in der zweiten Synthesestufe zu produzierenden Produktproteins, beispielsweise ein Gen für ein Chaperon (löslichkeitsfördernd für das Produktprotein) eingesetzt. Das Chaperon Gen steht unter der Kontrolle des E. coli Promotors. Nach Abschluss der ersten Synthesestufe wird eine Recyclingstufe durchgeführt, wobei eine Abtrennung des Expressionsproduktes (Chaperon) nicht erfolgt, sondern lediglich eine Supplementierung sowie die Zugabe eines unter der Kontrolle des T7 Promotors stehenden Gens für das Produktprotein. Des weiteren wird ein Inhibitor der E. coli RNA Polymerase, beispielsweise Rifampicin, zugegeben. In der zweiten Synthesestufe erfolgt daher praktisch ausschließlich die Expression des Produktproteins, wobei dieses aufgrund der Anwesenheit der Chaperone aus der ersten Synthesestufe mit erheblich verbesserter Löslichkeit anfällt. Alternativ zur Inhibierung einer in der ersten Synthesestufe verwendeten RNA Polymerase kann das in dieser Stufe eingesetzte Gen bzw. Templat für die zweite Synthesestufe in der Konzentration reduziert werden, sei es durch Abtrennung, sei es durch Verdünnung.

### Beispiel 5: Apparatur für ein erfindungsgemäßes Verfahren

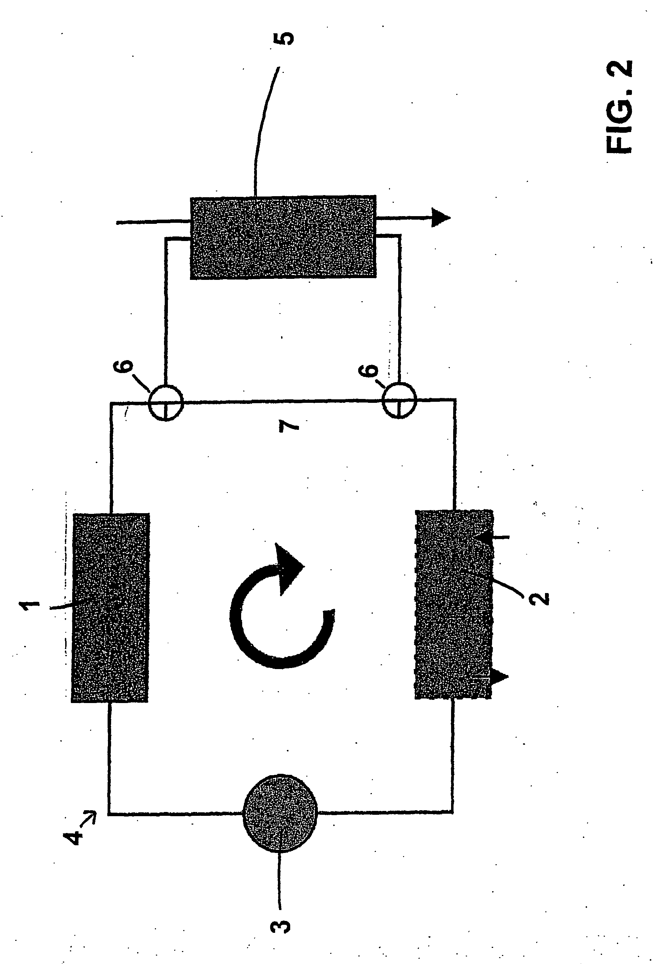

Figur 2 zeigt eine für Erfindung geeignete Apparatur. Man erkennt ein Reaktionsmodul 1, ein Recyclingmodul 2, Mittel 3 zur Förderung von Lösungen sowie eine Kreislaufleitung 4. In dem Reaktionsmodul 1 werden die Stufen b), b') und/oder e') durchgeführt. In dem Recyclingmodul 2 erfolgen die Stufen c), d), c') und/oder d'). Die Mittel 3 zur Förderung von Lösungen werden dabei mit der Maßgabe angesteuert, dass die erfindungsgemäßen Verfahrenstufen nach den definierten Zeiträumen aufeinander folgen. Man erkennt des weiteren ein Abtrennungsmodul 5, in welchem Expressionprodukt aus der Lösung abtrennbar ist. Es sind Umschaltmittel 6 vorgesehen, welche das Abtrennungsmodul 5 in die Kreislaufleitung 4 an Stelle des Bypass 7 einschalten.

## Patentansprüche

1. Verfahren zur präparativen in vitro Synthese eines Expressionsproduktes in einem zellfreien Transkriptions-/Translationssystem mit den folgenden Verfahrensstufen:
a) es wird in einem Reaktionsgefäß eine Reaktionslösung mit den folgenden Synthesesubstanzen angesetzt: Komponenten des Transkriptions-/Translationsapparates für ein definiertes Expressionsprodukt, Aminosäuren, sowie Energie liefernde und für die Synthese des definierten Proteins notwendige Stoffwechselbestandteile,
b) in dem Reaktionsgefäß wird die Synthese in einem definierten Zeitraum durchgeführt, und zwar ohne Abtrennung von sich bildenden Substanzen und ohne Zufuhr von sich verbrauchenden Synthesesubstanzen innerhalb des definierten Zeitraumes,
c) nach Ablauf des definierten Zeitraumes wird die Reaktionslösung einer Abtrennungsverfahrensstufe unterworfen, in welcher gebildete niedermolekulare Stoffwechselprodukte und/oder Reaktionsinhibitoren aus der Lösung abgetrennt werden,
d) unmittelbar vor, nach oder zugleich mit Stufe c) werden verbrauchte Synthesesubstanzen supplementiert,
e) die Stufen b), c) und d) werden mit der Reaktionslösung aus Stufe d) zumindest einmal wiederholt, wobei bei letztmaliger Ausführung der Stufe b) die Stufen c) und d) entfallen können.

2. Verfahren nach Anspruch 1, wobei in der Stufe c) und/oder anschließend an Stufe e) Expressionsprodukte aus der Lösung abgetrennt werden.

3. Verfahren nach Anspruch 1 oder 2, wobei die Stufen b), c) und d) einmal bis zehnmal, vorzugsweise einmal bis fünfmal wiederholt werden.

4. Verfahren nach einem der Ansprüche 1 bis 3, wobei der definierte Zeitraum zwischen 0,1 bis 10 Stunden, vorzugsweise zwischen 0,5 bis 3 Stunden, beträgt.

5. Verfahren nach einem der Ansprüche 1 bis 4, wobei die Stufe c) mittels Gelfiltration, Ultrafiltration, Dialyse, Diafiltration oder Matrices mit selektiven Bindungseigenschaften für niedermolekulare Stoffwechselprodukte und/oder Reaktionsinhibitoren durchgeführt wird.

6. Verfahren zur präparativen in vitro Proteinsynthese in einem zellfreien Transkriptions-/Translationssystem mit den folgenden Verfahrenstufen:
a') es wird in einem Reaktionsgefäß eine Reaktionslösung mit den folgenden Synthesesubstanzen angesetzt: Komponenten des Transkriptions-/Translationsapparates für ein definiertes erstes Expressionsprodukt, optional Komponenten des Transkriptions-/Translationsapparates für ein definiertes von dem ersten Expressionsprodukt verschiedenes zweites Expressionsprodukt, Aminosäuren, sowie Energie liefernde und für die Synthese notwendige Stoffwechselbestandteile,
b') in dem Reaktionsgefäß wird die Synthese des ersten Proteins in einem ersten definierten Zeitraum durchgeführt, und zwar ohne Zufuhr von sich verbrauchenden Synthesesubstanzen innerhalb des ersten definierten Zeitraumes,
c') optional werden gebildete niedermolekulare Stoffwechselprodukte aus der Lösung abgetrennt,
d') nach Ablauf des definierten Zeitraumes wird die Reaktionslösung mit verbrauchten Synthesesubstanzen supplementiert und, sofern nicht bereits in Stufe a) zugesetzt, mit Komponenten des Transkriptions-/Translationsapparates für das definierte zweite Expressionsprodukt versetzt,
e') in dem Reaktionsgefäß wird die Synthese des zweiten Proteins in einem definierten zweiten Zeitraum durchgeführt, und zwar ohne Abtrennung von sich bildenden Substanzen und ohne Zufuhr von sich verbrauchenden Synthesesubstanzen innerhalb des definierten Zeitraumes.

7. Verfahren nach Anspruch 6, wobei anschließend an Stufe e') das Verfahren nach einem der Ansprüche 1 bis 5, beginnend mit der Stufe c), durchgeführt wird.

8. Verfahren nach einem der Ansprüche 6 oder 7, wobei die Transkriptions-/Translationsapparate für das erste Expressionsprodukt und das zweite Expressionsprodukt verschiedene erste und zweite regulatorische Sequenzen enthalten, wobei eine für das erste Expressionsprodukt codierende erste Gensequenz unter der Kontrolle der ersten regulatorischen Sequenz und eine für das zweite Expressionsprodukt codierende zweite Gensequenz unter der Kontrolle der zweiten regulatorischen Sequenz stehen.

9. Verfahren nach einem der Ansprüche 6 bis 8 in der Ausführungsform mit Komponenten des Transkriptions-/Translationsapparates für ein definiertes von dem ersten Expressionsprodukt verschiedenes zweites Expressionsprodukt in Stufe a'), wobei in der Stufe b') die zweite regulatorische Sequenz inhibiert wird, und wobei in der Stufe e') die erste regulatorische Sequenz inhibiert wird.

10. Verfahren nach einem der Ansprüche 6 bis 8 in der Ausführungsform mit Zugabe der Komponenten des Transkriptions-/Translationsapparat für das definierte zweite Expressionsprodukt in Stufe d'), wobei in der Stufe e') die erste regulatorische Sequenz inhibiert wird.

## Claims

1. A method for the preparative in vitro synthesis of an expression product in a cell-free transcription/translation system comprising the following steps:
a) in a reaction vessel, a reaction solution with the following synthesis substances is prepared: components of the transcription/translation apparatus for a defined expression product, amino acids, and metabolic components supplying energy and being necessary for the synthesis of the defined protein,
b) in the reaction vessel, the synthesis is carried out in a defined period of time, without separation of forming substances and without addition of self-consuming synthesis substances within the defined period of time,
c) after expiration of the defined period of time, the reaction solution is subjected to a separation step, in which formed low-molecular metabolic products and/or reaction inhibitors are separated from the solution,
d) immediately before, after or at the same time as step c), consumed synthesis substances are supplemented,
e) steps b), c) and d) are repeated at least once with the reaction solution of step d), and in the last execution of step b), steps c) and d) can be omitted.

2. The method according to claim 1, wherein in step c) and/or following step e), expression products are separated from the solution.

3. The method according to claim 1 or 2, wherein steps b), c) and d) are repeated one to ten times, preferably one to five times.

4. The method according to one of claims 1 to 3, wherein the defined period of time is between 0.1 and 10 hours, preferably between 0.5 and 3 hours.

5. The method according to one of claims 1 to 4, wherein step c) is carried out by means of gel filtration, ultrafiltration, dialysis, diafiltration or matrices with selective binding properties for low-molecular metabolic products and/or reaction inhibitors.

6. A method for the preparative in vitro protein synthesis in a cell-free transcription / translation system comprising the following steps:
a') in a reaction vessel, a reaction solution with the following synthesis substances is prepared: components of the transcription/translation apparatus for a defined first expression product, optionally components of the transcription/translation apparatus for a defined second expression product being different from the first expression product, amino acids, and metabolic components supplying energy and being necessary for the synthesis,
b') in the reaction vessel, the synthesis of the first protein is carried out in a first defined period of time, without addition of self-consuming synthesis substances within the first defined period of time,
c') optionally, formed low-molecular metabolic products are separated from the solution,
d') after expiration of the defined period of time, the reaction solution is supplemented with consumed synthesis substances and, if not added in step a) already, reacted with components of the transcription/translation apparatus for the defined second expression product,
e') in the reaction vessel, the synthesis of the second protein is carried out in a defined second period of time, without separation of forming substances and without addition of self-consuming synthesis substances within the defined period of time.

7. The method according to claim 6, wherein following step e') the method according to one of claims 1 to 5 is carried out, beginning with step c).

8. The method according to one of claims 6 or 7, wherein the transcription/translation apparatuses for the first expression product and the second expression product comprise different first and second regulatory sequences, wherein a first gene sequence coding for the first expression product is under control of the first regulatory sequence and a second gene sequence coding for the second expression product is under control of the second regulatory sequence.

9. The method according to one of claims 6 to 8 in the embodiment comprising components of the transcription/translation apparatus for a defined second expression product being different from the first expression product in step a'), wherein in step b') the second regulatory sequence is inhibited, and wherein in step e') the first regulatory sequence is inhibited.

10. The method according to one of claims 6 to 8 in the embodiment comprising the addition of the components of the transcription/translation apparatus for the defined second expression product in step d'), wherein in step e') the first regulatory sequence is inhibited.

## Revendications

1. Procédé pour la synthèse in vitro préparative d'un produit d'expression dans un système de transcription/traduction sans cellules comprenant les étapes suivantes:
a) dans un réacteur, une solution de réaction avec les substances de synthèse suivantes est préparée: des composants de l'appareil de transcription/traduction pour un produit d'expression défini, des acides aminés, et des composants métaboliques fournissant de l'énergie et étant nécessaires pour la synthèse de la protéine définie,
b) dans le réacteur, la synthèse est effectuée dans une durée défini, sans séparation de substances se formant et sans addition de substances de synthèse d'autoconsommation au-dedans de la durée définie,
c) après l'expiration de la durée définie, la solution de réaction est soumise à une étape de séparation, dans laquelle des produits métaboliques à bas poids moléculaire se formant et/ou des inhibiteurs de réaction sont séparés de la solution,
d) immédiatement avant, après ou au même temps comme l'étape c), des substances de synthèse consommées sont supplémentées,
e) les étapes b), c) et d) sont répétées au moins une fois avec la solution de réaction de l'étape d), et dans la dernière exécution de l'étape b), les étapes c) et d) peuvent être omises.

2. Procédé selon la revendication 1, dans lequel dans l'étape c) et/ou après l'étape e), des produits d'expression sont séparés de la solution.

3. Procédé selon la revendication 1 ou 2, dans lequel les étapes b), c) et d) sont répétées une à dix fois, de préférence une à cinq fois.

4. Procédé selon une des revendications 1 à 3, dans lequel la durée définie est entre 0,1 et 10 heures, de préférence entre 0,5 et 3 heures.

5. Procédé selon une des revendications 1 à 4, dans lequel l'étape c) est effectuée au moyen de filtration sur gel, ultrafiltration, dialyse, diafiltration ou matrices avec des propriétés de liage sélectives pour des produits métaboliques à bas poids moléculaire et/ou des inhibiteurs de réaction.

6. Procédé pour la synthèse de protéines in vitro préparative dans un système de transcription/traduction sans cellules comprenant les étapes suivantes:
a') dans un réacteur, une solution de réaction avec les substances de synthèse suivantes est préparée: des composants de l'appareil de transcription/traduction pour un premier produit d'expression défini, optionnellement des composants de l'appareil de transcription/traduction pour un deuxième produit d'expression défini étant différent du premier produit d'expression, des acides aminés, et des composants métaboliques fournissant de l'énergie et étant nécessaires pour la synthèse,
b') dans le réacteur, la synthèse de la première protéine est effectuée dans une première durée définie, sans addition de substances de synthèse d'autoconsommation au-dedans de la première durée définie,
c') optionnellement, des produits métaboliques à bas poids moléculaire se formant sont séparés de la solution,
d') après l'expiration de la durée définie, la solution de réaction est supplémentées avec des substances de synthèse consommées et, si pas ajoutés déjà dans l'étape a), réagie avec des composants de l'appareil de transcription/traduction pour le deuxième produit d'expression défini,
e') dans le réacteur, la synthèse de la deuxième protéine est effectuée dans une deuxième durée définie, sans séparation de substances se formant et sans addition de substances de synthèse d'autoconsommation au-dedans de la durée définie.

7. Procédé selon la revendication 6, dans lequel après l'étape e'), le procédé selon une des revendications 1 à 5 est effectué, commençant avec l'étape c).

8. Procédé selon une des revendications 6 ou 7, dans lequel les appareils de transcription/traduction pour le premier produit d'expression et le deuxième produit d'expression comprennent des premières et deuxièmes séquences régulatrices différentes, dans lequel une première séquence génétique codant pour le premier produit d'expression est sous contrôle de la première séquence régulatrice et une deuxième séquence génétique codant pour le deuxième produit d'expression est sous contrôle de la deuxième séquence régulatrice.

9. Procédé selon une des revendications 6 à 8 dans la forme d'exécution comprenant des composants de l'appareil de transcription/traduction pour un deuxième produit d'expression défini étant différent du premier produit d'expression dans l'étape a'), dans lequel dans l'étape b') la deuxième séquence régulatrice est inhibée, et dans lequel dans l'étape e') la première séquence régulatrice est inhibée.

10. Procédé selon une des revendications 6 à 8 dans la forme d'exécution comprenant l'addition des composants de l'appareil de transcription / traduction pour le deuxième produit d'expression défini dans l'étape d'), dans lequel dans étape e') la première séquence régulatrice est inhibée.
